# EUROPEAN PATENT APPLICATION

(11) **EP 4 768 578 A1**
(43) Date of publication of application: **01.07.2026**
(21) Application number: 23949796.9
(22) Date of filing: 24.08.2023
(51) Int. Cl.: C12N 15/09, C12N 15/113, A61K 31/7088, A61P 29/00

(54) **BISPECIFIC NUCLEIC ACID**

(71) Applicant: Dual Targets, Inc., Minato-ku, Tokyo 105-0013 (JP)
(72) Inventor: YONEYAMA, Hiroyuki, Tokyo 105-0013 (JP)
(74) Representative: Grünecker Patent- und Rechtsanwälte PartG mbB
(86) International application number: PCT/JP2023/030552
(87) International publication number: WO 2025/041340

(57) **Abstract**

The present invention provides a bispecific nucleic acid molecule comprising an antisense strand having a sequence that specifically binds to CHST2 and also specifically binds to CHST4.

## Description

### TECHNICAL FIELD

The present invention relates to a bispecific nucleic acid molecule, particularly a nucleic acid molecule having bispecificity for carbohydrate sulfotransferases CHST2 and CHST4, a pharmaceutical composition containing the bispecific nucleic acid molecule, and the use thereof.

### BACKGROUND ART

Chronic inflammation is a persistent and often irreversible reaction to various external insults, causing tissue destruction, functional impairment, and progressive clinical symptoms in all organs throughout the body. In chronically inflamed tissues, infiltration of leukocytes, which governs inflammation and immunity, is characteristic and is considered to be a major cause of exacerbating and perpetuating lesions associated with functional impairment. Although capillaries are the starting point of inflammation, particularly in chronic inflammation, the phenomenon in which characteristic vascular endothelium newly emerges at the local site of inflammation has been observed and reported. Under physiological conditions, these vessels are only expressed in "lymphoid tissues" such as lymph nodes and the spleen, and they are high endothelial venule (HEV, hereinafter described as "HEV")-like vessels that serve as entry sites for lymphocyte "homing" (the phenomenon where lymphocytes in the blood migrate from blood vessels into lymphoid tissues in the recirculation of lymphocytes). Since it has been reported in many cases of chronic inflammation and allergic diseases such as bronchial asthma, allergic rhinitis, contact dermatitis, chronic rheumatoid arthritis, and inflammatory bowel disease, it is considered to be a phenomenon that is not specific to an organ or disease, but is rather a widely common in chronic inflammation induced in any tissue. Therefore, it is suggested to be involved in the recruitment of leukocytes to sites of inflammatory infiltration in chronic inflammation and allergic diseases, as well as in the pathogenesis, functional impairment, and clinical symptoms of chronic lesions, and is expected to be applied to the development of novel therapeutic approaches (References 1 to 6).

The vascular endothelial cells of HEV express a characteristic sulfated glycan called sialyl 6-sulfo Lewis X (S-sLe^{x}), which serves as a ligand for L-selectin (CD62L) on the surface of leukocytes. For the purpose of treating chronic inflammation, research and development aimed at suppressing inflammatory cell infiltration by inhibiting L-selectin on the leukocyte-side or S-sLe^{x} on the HEV-side has been conducted for some time, but no inhibitors have yet emerged for practical clinical application by now. In particular, antibodies such as MEL-14, which inhibit L-selectin on the leukocyte-side, have been developed, and in non-clinical level, these antibodies were confirmed to inhibit the "rolling" function, which is an early stage of cell extravasation, and thereby inhibiting cell infiltration. Therefore, there is significant expectation for antibody therapeutics. In particular, in antibody therapeutics administered via systemic circulation (bloodstream), it is considered that targeting leukocytes present in the circulating bloodstream is highly effective because they can act more directly compared to vascular endothelial cells distributed in tissues. However, since the major cell population expressing L-selectin is naive lymphocytes, it was considered that inhibiting the circulation of naive lymphocytes could induce severe immune functional impairment. That is, safety issues remained unresolved for systemically administered pharmaceuticals, which include antibody therapeutics, making approaches targeting HEV ligand molecules difficult to put into clinical application (References 1-8).

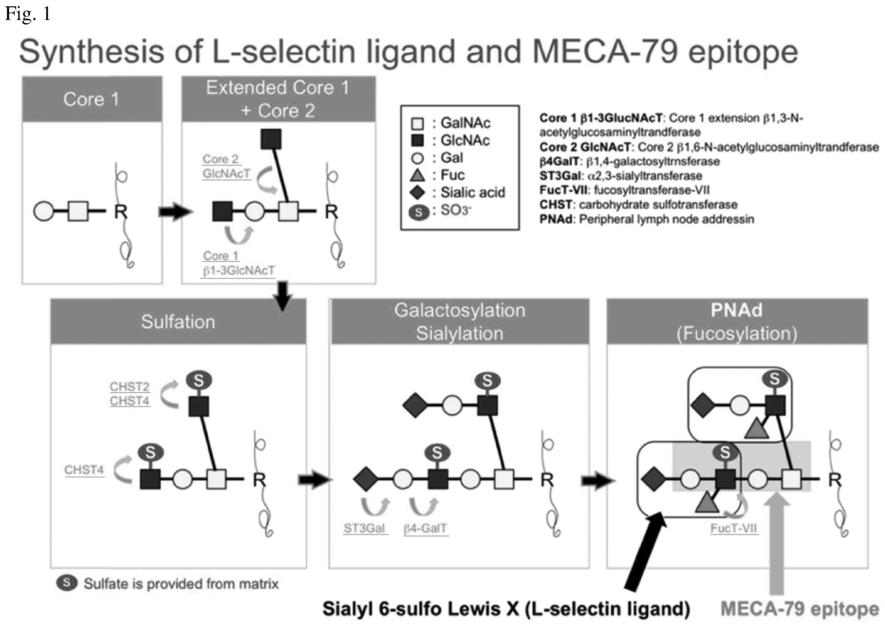

On the other hand, it is considered that the approach of inhibiting molecules on the HEV-side has the advantage of potentially lower safety concerns, particularly in chronic inflammation, as confirmed by the phenomenon of new (de novo) emergence of HEV-like blood vessels at the site of inflammation. The antibody "MECA-79" has been identified as recognizing HEV, and this is an antibody recognizing the sialomucin glycoprotein Peripheral node addressin (hereinafter PNAd) expressed on HEV and is the most widely used as an antibody for detecting HEV via immunohistochemical staining (References 9 to 11). The antigenic determinant of the MECA-79 antibody is an O-linked glycan attached to core proteins (CD34, Podocalyxin, GlyCAM-1, Endomucin, and Nepmucin have been identified to date), and this glycan consists of a core-1 structure (N-acetylgalactosamine (GalNAc) residue + galactose (Gal) residue) with an S-sLe^{x} structure attached (Fig. 1). In nonclinical studies, the MECA-79 antibodies have been shown to be effective in suppressing leukocyte infiltration and treating asthma models, and thus suggesting therapeutic potential targeting PNAd. However, the subclass of the MECA-79 antibody belongs to the IgM, as is the case with many antibodies to glycans. Although this subclass is useful as a research tool or diagnostic agent, this made it unsuitable for formulation and clinical application as a therapeutic drug. In addition, while there are reports that antibodies recognizing the S-sLe^{x} structure have shown efficacy in research tools and animal experimental models, the glycan structures and states of addition do not necessarily match between humans and animals, so that the drug efficacy is likely to be very heterogeneous. Since the sLe^{x} structure is also expressed on leukocyte surfaces, safety concerns remain, similar to those with antibodies against L-selectin, and it is therefore considered unsuitable for clinical realization as a pharmaceutical (References 1 to 17).

To the contrary, as the process by which PNAd (L-selectin ligand glycans) is synthesized has been elucidated (Fig. 1), an approach can be considered to inhibit PNAd synthesis, particularly the synthesis of de novo PNAd that newly emerges with inflammation by effectively inhibiting some step in these synthesis processes. Synthesis involves four major steps of extension of the core 1 structure and branching of the core 2 structure, sulfation, sialylation, and fucosylation, and specific enzymes responsible for adding glycans at each step have been identified (underlined words in Fig. 1). According to the previous reports, it has been found that Carbohydrate sulfotransferase 2: CHST2 (alternative name: N-acetylglucosamine-6-sulfotransferase 1: GlcNAc6ST-1), which is a sulfotransferase involved in sulfation, is responsible for 6-sulfation of GlcNAc residues on core 2-branched chains, and similarly CHST4 (alternative name: GlcNAc6ST-2) is responsible for 6-sulfation on both core 1 extension and core 2-branched chains (References 9 to 11). From analysis of knockout mice for these genes, it was revealed that simultaneous knockdown of both genes, rather than either alone, strongly suppressed MECA-79 expression and suppressed lymphocyte infiltration (References 1, 4, 5 and 17).

Regarding inhibitors having bispecificity, research and development of bispecific antibodies has been active in recent years, and they have already been clinically implemented. On the other hand, CHST2 and CHST4 are Golgi proteins, and in vivo, administering antibodies (anti-CHST2 antibodies and anti-CHST4 antibodies) from outside the cell via routes such as the bloodstream does not result in the exertion of neutralizing activity functions, making antibody therapeutics impractical. Further, in creating small molecule inhibitors targeting glycosyltransferases, obtaining compounds with high specificity has been extremely difficult. It has been impossible to improve the IC50 to the level typically seen in clinically applied small molecule compounds, and it was difficult to advance such compounds to the development stage as drug candidates. Under such circumstances, the present inventors pursued an approach targeting nucleic acids (RNA) using nucleic acid therapeutics, particularly antisense nucleic acid (Antisense Oligonucleotides; ASO) utilizing single-stranded RNA or siRNA utilizing double-stranded RNA (References 4, 5, 18 and 19).

Nucleic acid therapeutics (ASOs and siRNAs) exert their effects by specifically binding to the specific base sequences of their target mRNAs, and therefore this has positioned them as a novel therapeutic modality with exceptionally high specificity, and has been rapidly advancing to clinical application. They hold great promise for targeting molecules that could not be reached by conventional small molecule compounds or antibody therapeutics, so that it is expected that any disease-related molecule can be targeted as a therapeutic target. The concept of designing a nucleic acid therapeutic with high specificity that binds to two mRNAs simultaneously with a single nucleic acid therapeutic fundamentally contradicts Watson-Crick pairing rules, and thus such an idea has not been considered in the medical biology field (Reference 20). However, as the popularity of bispecific antibodies demonstrates, there are actually numerous disease states where targeting two different mRNAs offers greater therapeutic potential. Therefore, when targeting of different two, or two or more mRNAs, basic research has been conducted on using nucleic acid therapeutics as a cocktail, that is, when using siRNA, administering a cocktail of two different siRNAs linked together (References 18 and 19). It can be considered that advancing cocktail therapies toward clinical practicality faces significant challenges, beyond the straightforward cost increase due to the greater number of bases, there is a synergistic rise in development costs associated with expanded manufacturing processes and toxicity testing items, additionally, the risk of discontinuation caused by toxicity, and, in vivo, it is practically difficult for the components to be taken up into the same cell at exactly the same time, whereby this leads to a high risk of instability and uncertainty in therapeutic efficacy.

### PRIOR ART DOCUMENTS

### PATENT DOCUMENTS

Patent Document 1: PCT/US93/11612
Patent Document 2: PCT/US95/05463
Patent Document 3: PCT/US2020/017879
Patent Document 4: PCT/KR2021/003488

### NON-PATENT DOCUMENTS

1) Blanchard L, Girard JP. High endothelial venules (HEVs) in immunity, inflammation and cancer. Angiogenesis. 24:719-753. 2021.
2) Kogame T, Kabashima K, Egawa G. Putative Immunological Functions of Inducible Skin-Associated Lymphoid Tissue in the Context of Mucosa-Associated Lymphoid Tissue. Front Immunol. 12:733484. 2021.
3) Yoneyama H, Matsuno K, Zhang Y, Murai M, Itakura M, Ishikawa S, Hasegawa G, Naito M, Asakura H, Matsushima K. Regulation by chemokines of circulating dendritic cell precursors, and the formation of portal tract-associated lymphoid tissue, in a granulomatous liver disease. J Exp Med. 193: 35-49, 2001.
4) Kawashima H. Roles of sulfated glycans in lymphocyte homing. Biol Pharm Bull. 29: 2343-2349, 2006.
5) Kenji Uchimura. Cell Surface Sulfated Glycans Recognized by the "Homing Receptor" L-Selectin. Biochemistry 85: 244-252, 2013.
6) van Zante A, Rosen SD. Sulphated endothelial ligands for L-selectin in lymphocyte homing and inflammation. Biochem Soc Trans. 31: 313-3187, 2003.
7) Ley K. The role of selectins in inflammation and disease. Trends Mol Med. 9: 263-268, 2003.
8) Smith BAH, Bertozzi CR. The clinical impact of glycobiology: targeting selectins, Siglecs and mammalian glycans. Nat Rev Drug Discov. 20: 217-243, 2021.
9) Streeter PR, Rouse BT, Butcher EC. Immunohistologic and functional characterization of a vascular addressin involved in lymphocyte homing into peripheral lymph nodes. J Cell Biol. 107: 1853-1862, 1998.
10) Yeh JC, Hiraoka N, Petryniak B, Nakayama J, Ellies LG, Rabuka D, Hindsgaul O, Marth JD, Lowe JB, Fukuda M. Novel sulfated lymphocyte homing receptors and their control by a Core1 extension beta 1,3-N-acetylglucosaminyltransferase. Cell. 105: 957-969, 2001.
11) Michie SA, Streeter PR, Bolt PA, Butcher EC, Picker LJ. The human peripheral lymph node vascular addressin. An inducible endothelial antigen involved in lymphocyte homing. Am J Pathol. 143: 1688-1698, 1993.
12) Lechleitner S, Kunstfeld R, Messeritsch-Fanta C, Wolff K, Petzelbauer P. Peripheral lymph node addressins are expressed on skin endothelial cells. J Invest Dermatol. 113: 410-414, 1999.
13) Yang J, Rosen SD, Bendele P, Hemmerich S. Induction of PNAd and N-acetylglucosamine 6-O-sulfotransferases 1 and 2 in mouse collagen-induced arthritis. BMC Immunol. 7:12, 2006.
14) Rosen SD, Tsay D, Singer MS, Hemmerich S, Abraham WM. Therapeutic targeting of endothelial ligands for L-selectin (PNAd) in a sheep model of asthma. Am J Pathol. 166: 935-944, 2005.
15) Matsumura R, Hirakawa J, Sato K, Ikeda T, Nagai M, Fukuda M, Imai Y, Kawashima H. Novel Antibodies Reactive with Sialyl Lewis X in Both Humans and Mice Define Its Critical Role in Leukocyte Trafficking and Contact Hypersensitivity Responses. J Biol Chem. 290: 15313-15326, 2015.
16) Xiong W, Liu W, Nishida S, Komiyama D, Liu W, Hirakawa J, Kawashima H. Therapeutic Effects of an Anti-sialyl Lewis X Antibody in a Murine Model of Allergic Asthma. Int J Mol Sci. 22: 9961, 2021.
17) Ohmichi Y, Hirakawa J, Imai Y, Fukuda M, Kawashima H. Essential role of peripheral node addressin in lymphocyte homing to nasal-associated lymphoid tissues and allergic immune responses. J Exp Med. ;208: 1015-1025, 2011.
18) Friedrich M, Aigner A. Therapeutic siRNA: State-of-the-Art and Future Perspectives. BioDrugs. 36: 549-571, 2022.
19) Michael FM, Chandran P, Chandramohan K, Iyer K, Jayaraj K, Sundaramoorthy R, Venkatachalam S. Prospects of siRNA cocktails as tools for modifying multiple gene targets in the injured spinal cord. Exp Biol Med (Maywood). 244: 1096-1110, 2019.
20) Kondo J, Westhof E. Base pairs and pseudo pairs observed in RNA-ligand complexes. J Mol Recognit. 23: 241-252, 2010.

### SUMMARY OF THE INVENTION

### PROBLEM TO BE SOLVED BY THE INVENTION

The present invention aims to provide a novel therapeutic method and therapeutic agent that improves lesions by controlling leukocyte infiltration through suppression of HEV-like blood vessels locally expressed with inflammation, which has traditionally been difficult to suppress infiltration of leukocytes into lesions, particularly chronic lesions, in various inflammatory or allergic diseases.

### MEANS TO SOLVE THE PROBLEM

In order to inhibit the synthesis of the glycoprotein structure (PNAd) characteristic of HEV-like blood vessels, the present inventors attempted to create a nucleic acid compound (bispecific nucleic acid) that simultaneously hybridizes to both human CHST2 mRNA and CHST4 mRNA, which are molecules responsible for the sulfation process in the PNAd synthesis process. As a result, they found that it is possible to design nucleic acid compounds that hybridize to both CHST2 mRNA and CHST4 mRNA by replacing part of the nucleic acid compound's base sequence with artificial bases instead of the original C (cytidylic acid), A (adenylic acid), U (uridylic acid) and G (guanylic acid). Also, the sequences of CHST2 mRNA and CHST4 mRNA targeted by the nucleic acid compounds of the present invention (the regions to which these nucleic acid compounds bind) are identical in humans, mice, rats, monkeys, dogs and cats, and therefore could be designed without considering differences between these species, particularly between mammalian species.

More specifically, the present invention provides the following [1] to [10].
[1] A bispecific nucleic acid molecule comprising an antisense strand having a sequence that specifically binds to CHST2 and also specifically binds to CHST4.
[2] The bispecific nucleic acid molecule described in [1], which is an siRNA or an antisense oligonucleotide.
[3] The bispecific nucleic acid molecule described in [2], wherein the bispecific nucleic acid molecule is the siRNA having a sequence shown in SEQ ID NOs: 5 and 6, SEQ ID NOs: 7 and 8, SEQ ID NOs: 9 and 10, SEQ ID NOs: 11 and 12, or SEQ ID NOs: 13 and 14, or is the antisense oligonucleotide having any of SEQ ID NOs: 25 to 29.
[4] The bispecific nucleic acid molecule described in [2] or [3], which comprises an artificial base.
[5] The bispecific nucleic acid molecule described in [4], which comprises a chemically modified artificial base.
[6] A pharmaceutical composition comprising the bispecific nucleic acid molecule described in any one of [1] to [5].
[7] The pharmaceutical composition described in [6] for preventing or treating an inflammatory or allergic disease.
[8] Use of the bispecific nucleic acid molecule described in any one of [1] to [5] for the manufacture of a pharmaceutical composition for preventing or treating an inflammatory or allergic disease.
[9] A method for preventing or treating patients with an inflammatory or allergic disease, comprising administering the pharmaceutical composition described in [6] or [7] to a patient in need of such treatment.

### EFFECT OF THE INVENTION

The bispecific nucleic acid molecules of the present invention can be artificially synthesized. The inventors demonstrated that, in human cells, the bispecific nucleic acid molecules of the present invention dose-dependently suppress the expression of CHST2 mRNA and CHST4 mRNA and suppress the expression of MECA-79, which recognizes the glycan structure of PNAd. It is noteworthy that, the bispecific nucleic acid molecules of the present invention use artificial bases in part thereof, but they suppressed the dual targets without inducing non-specific inflammatory reactions such as interferon responses or TLR responses. Further, in a mouse model of atopic dermatitis, it was demonstrated that administration of the bispecific nucleic acid molecule of the present invention resulted in a significant reduction in MECA-79-positive HEV-like blood vessels and a concomitant significant suppression of inflammatory lymphocyte infiltration, and also acts its efficacy in vivo.

According to the present invention, against the formation of leukocyte infiltrated lesions, which is a characteristic of a feature of various chronic inflammation and allergic diseases and the root cause of their intractability, that the lesions are improved by safely suppressing HEV-like blood vessels that locally express with inflammation, thereby providing novel therapeutic applications and clinical practical applications. Further, while simultaneous suppression of CHST2 and CHST4, which are the molecules synthesizing HEV-like blood vessels, within the same cell is essential for suppressing HEV-like blood vessel synthesis, the present nucleic acid compound is a bispecific nucleic acid that simultaneously suppresses CHST2/CHST4, so that it provides a novel therapy that improves inflammation by suppressing locally expressed HEV-like blood vessels.

### BRIEF DESCRIPTION OF THE DRAWINGS

Fig. 1 shows the synthesis process of the MECA-79 antibody epitope (sialomucin glycoprotein: PNAd) and the glycosyltransferase (green): The MECA-79 epitope (PNAd) is an O-linked glycan attached to core proteins (labeled "R" in the figure: CD34, Podpcalyxin, GlyCAM-1, Endomucin and Nepmucin have been identified to date), and is a structure where an S-sLe^{x} structure (enclosed in the figure) is attached to a core 1 structure (N-acetylgalactosamine (GalNAc) residue + galactose (Gal) residue). Synthesis involves roughly four major steps: extension of the core 1 structure, and branching, sulfation, sialylation and fucosylation of the core 2 structure, and specific enzymes responsible for adding glycans at each step have been identified (underlined words). The enzyme extending the core 1 structure (Core 1) by transferring N-acetylglucosamine (GlcNAc: square) is Core 1 b1-3GlcNAcT, while the enzyme producing the core 2 branch is Core 2 GlcNAcT (center top of figure). In the subsequent sulfation step (S: ellipse), CHST2 catalyzes 6-sulfation of the GlcNAc residue on the core 2 branching chain, while CHST4 catalyzes 6-sulfation on both the core 1 extension chain and the core 2 branching chain (bottom left of figure). The enzyme responsible for adding galactose (Gal: circle) to the sulfated N-acetylglucosamine (GlcNAc: square) is b4-GalT, and the enzyme responsible for sialylation (sialic acid: diamond-shaped transfer) of this galactose residue is STGal (center bottom of figure). Finally, the synthesis of the S-sLe^{x} structure is completed through the step of fucosylation (fucose: triangular transfer) (bottom right of figure). The enzymes involved in this step are FucT-IV and FucT-VII, with FucT-VII thought to play a larger role.
Fig. 2 shows the knockdown effect of bispecific nucleic acids (10 nucleic acids of DS01 to DS10) targeting human CHST2 or CHST4 on their respective target mRNAs (CHST2 mRNA and CHST4 mRNA).
Fig. 3 shows the stability of the binding complex between the antisense strand (single stranded RNA, SS06 to SS10) among DS06 to 10 and human CHST2 mRNA, indicated by the double-helix melting temperature (Tm).
Fig. 4 shows the stability of the binding complex between the antisense strand (single stranded RNA, SS06 to SS10) among DS06 to 10 and human CHST4 mRNA, indicated by the double-helix melting temperature (Tm).
Fig. 5 shows the dose-dependent knockdown effect of bispecific nucleic acids (2 nucleic acids of DS06 and DS10) targeting human CHST2 and CHST4 on their respective target mRNAs (CHST2 mRNA and CHST4 mRNA). Control: Vehicle (physiological saline) was used as the negative control, and PC: A cocktail of CHST2 siRNA + CHST4 siRNA was used as the positive control.
Fig. 6 shows the dose-dependent inhibitory effect of bispecific nucleic acids (2 nucleic acids of DS06 and DS10) targeting human CHST2 and CHST4 on MECA-79 expression. Control: Vehicle (physiological saline) was used as the negative control, and PC: A cocktail of CHST2 siRNA + CHST4 siRNA was used as the positive control.
Fig. 7 shows the effect of bispecific nucleic acid (DS06) targeting human CHST2 and CHST4 on interferon response gene (IFITM1) expression. Poly I:C is the positive control inducing interferon response.
Fig. 8 shows the effect of bispecific nucleic acid (DS06) targeting human CHST2 and CHST4 on TLR response. Control (positive control): Substances inducing each TLR response.
Fig. 9 shows the cumulative total amount of nucleic acid permeating artificial skin over time using DS06.
Fig. 10 shows the effect of the bispecific nucleic acid (DS06) targeting human CHST2 and CHST4 on mouse atopic dermatitis. After administering mite antigen to the ear auricle of NC/Nga mice (7 weeks old, male) to induce dermatitis, a DS-06-containing cream, a negative control cream, and tacrolimus ointment as a positive control were applied once daily for 20 days from day 11 to day 30, and the mice were slaughtered on day 31, and skin lesions were evaluated. HE staining findings.
Fig. 11 shows the effect (HEV-like blood vessels) of bispecific nucleic acid (DS06) targeting human CHST2 and CHST4 on mouse atopic dermatitis.
Fig. 12 shows the effect (T lymphocytes) of the bispecific nucleic acid (DS06) targeting human CHST2 and CHST4 on mouse atopic dermatitis.
Fig. 13 shows the effect (activated T lymphocytes) of the bispecific nucleic acid (DS06) targeting human CHST2 and CHST4 on mouse atopic dermatitis.
Fig. 14 shows the effect (regulatory T lymphocytes) of bispecific nucleic acid (DS06) targeting human CHST2 and CHST4 on mouse atopic dermatitis.
Fig. 15 shows the effect (IL-13 and IL-33) of bispecific nucleic acid (DS06) targeting human CHST2 and CHST4 on mouse atopic dermatitis.
Fig. 16 shows the effect (HEV-like blood vessels and T lymphocytes) of bispecific nucleic acid (DS06) targeting human CHST2 and CHST4 on mouse allergic rhinitis.

### EMBODIMENT OF THE INVENTION

Hereinafter the present invention will be described in detail.

The present invention relates to a bispecific nucleic acid molecule comprising an antisense strand having a sequence that specifically binds to CHST2 and also specifically binds to CHST4.

The CHST2 gene and CHST4 gene in the present invention are not particularly limited, but are typically derived from animals, more preferably from mammalians, and most preferably from humans. The CHST2 of the present invention is, as another name, also called as GlcNAc6ST-1 (N-acetylglucosamine 6-O-sulfotransferase-1), and the CHST4 is, as another name, also called as GlcNAc6ST-2 (N-acetylglucosamine 6-O-sulfotransferase-2).

The sequences of CHST2 (GlcNAc6ST1) and CHST4 (GlcNAc6ST-2) in the present invention can be obtained, for example, based on accession numbers NM_004267.4 and NM_001166395.1. As an example, the nucleotide sequence of the CHST2 gene in the present invention is described in SEQ ID NO: 1, and the amino acid sequence encoded by this gene is described in SEQ ID NO: 2. Also, the nucleotide sequence of the CHST4 gene is described in SEQ ID NO: 3, and the amino acid sequence encoded by this gene is described in SEQ ID NO: 4. Proteins consisting of amino acid sequences other than those above, having high identity, 70% or more, preferably 80% or more, more preferably 90% or more, and most preferably 95% or more, with the sequences shown in, for example, SEQ ID NO: 2 or SEQ ID NO: 4, and having the functions of the above proteins, are also included within the CHST2 protein and CHST4 protein of the present invention. Further, the CHST2 protein or CHST4 protein of the present invention may be, for example, a protein consisting of an amino acid sequence in which one or more amino acids are added, deleted, substituted, or inserted in the amino acid sequence shown in SEQ ID NO: 2 or SEQ ID NO: 4, wherein the number of amino acids that are typically altered is within 30 amino acids, preferably within 10 amino acids, more preferably within 5 amino acids, and most preferably within 3 amino acids.

The CHST2 gene or CHST4 gene of the present invention includes endogenous genes in other organisms (such as homologs of the above human genes) corresponding to DNA consisting of the nucleotide sequences shown, for example, in SEQ ID NO: 1 or SEQ ID NO: 3. Furthermore, endogenous DNA in other organisms corresponding to DNA consisting of the nucleotide sequences shown in SEQ ID NO: 1 or SEQ ID NO: 3 generally has high identity (homology) with the DNA shown in SEQ ID NO: 1 or SEQ ID NO: 3, respectively. High identity means preferably 70% or more, more preferably 80% or more, and most preferably 90% or more (e.g., 95% or more, further 96%, 97%, 98%, or 99% or more) homology. This homology is determined using the mBLAST algorithm (Altschul et al. (1990) Proc. Natl. Acad. Sci. USA 87: 2264-8; Karlin and Altschul (1993) Proc. Natl. Acad. Sci. USA 90: 5873-7). Further, said DNA is considered to hybridize with the DNA shown in SEQ ID NO: 1 or SEQ ID NO: 3, respectively, under stringent conditions when isolated from a biological organism. Here, "stringent conditions" may include, for example, "2×SSC, 0.1% SDS, 50°C," "2×SSC, 0.1% SDS, 42°C" and "1×SSC, 0.1% SDS, 37°C", and more stringent conditions of "2×SSC, 0.1% SDS, 65°C," "0.5×SSC, 0.1% SDS, 42°C" and "0.2×SSC, 0.1% SDS, 65°C".

The bispecific nucleic acid molecules of the present invention may be, for example, but are not limited to, siRNA, antisense oligonucleotides, miRNA, or decoys.

In the siRNA of the present invention, it may not necessarily be that all nucleotides are ribonucleotides (RNA). That is, in the present invention, one or several ribonucleotides constituting the siRNA may be corresponding deoxyribonucleotides, provided that the molecule itself has the function of suppressing the expression of the CHST2 gene and the CHST4 gene. This "corresponding" refers to the same base species (adenine, guanine, cytosine, thymine (uracil)), although the sugar moiety structure differs. For example, a deoxyribonucleotide corresponding to a ribonucleotide having adenine is a deoxyribonucleotide having adenine. Also, the term "several" is not particularly limited, but preferably refers to a small number, such as about 2 to 5.

The siRNA of the present invention can be readily prepared by a person skilled in the art using commercially available nucleic acid synthesizers. Also, for the synthesis of the desired RNA, general synthesis contract services can be utilized.

In the present specification, "siRNA suppressing expression of CHST2 gene" may also be expressed as "CHST2 siRNA," and "siRNA suppressing expression of CHST4 gene" may also be expressed as "CHST4 siRNA".

The bispecific nucleic acid molecules of the present invention may preferably be, but are not limited to, siRNA or antisense oligonucleotides having any of the following sequences.

### siRNA (DS01 to DS05):

In one embodiment, the bispecific nucleic acid molecules of the present invention may be siRNAs consisting of the antisense strand and sense strand shown in the following SEQ ID NOs: 5 to 24. Bases in the antisense strand marked with double underlines indicate mismatch base positions (where CHST2 and CHST4 do not match), for example, one or more positions selected from the group consisting of positions 3, 7, 11, 12, 13, 24 and 27 in SEQ ID NO: 5, positions 5, 8, 14, 16, 24, 26 and 27 in SEQ ID NO: 7, positions 9, 12, 18 and 20 in SEQ ID NO: 9, positions 8, 11, 17, 19 and 27 in SEQ ID NO: 11, positions 7, 10, 16, 18 and 26 in SEQ ID NO: 13, positions 3, 7, 11, 12, 13, 24 and 27 in SEQ ID NO: 15, positions 5, 8, 14, 16, 24, 26 and 27 in SEQ ID NO: 17, positions 9, 12, 18 and 20 in SEQ ID NO: 19, positions 8, 11, 17, 19 and 27 in SEQ ID NO: 21, and positions 7, 10, 16, 18 and 26 in SEQ ID NO: 23, may be modified, for example, but not limited to, 2'-OMe, 2'-MOE, or LNA modification. DNA is represented in bold. DS01 to DS05 have natural bases (G, A, U and C).

### [Chemical formula 1]

**DS01**
   **5' -ACGGGGGCUCGAGCCAGGUCCUCGUAGdTdT-3'** (SEQ ID **NO: 5:antisense)**
   **3' -dTdTUGCCCCCGAGCUCGGUCCAGGAGCAUC-5'** (SEQ ID **NO: 6:sense)**
**DS02**
   **5' -CCUCGUAGCGCACAAGCAGGUAGUGGUdTdT-3'** (SEQ ID NO: **7:antisense)**
   **3' -dTdTGGAGCAUCGCGUGUUCGUCCAUCACCA-5'** (SEQ ID NO: **8:sense)**
**DS03**
   **5' -AGGUCCUCGUAGCGCACAAGCAGGUAGATAT-3'** (SEQ ID **NO: 9:antisense)**
   **3' -dTdTUCCAGGAGCAUCGCGUGUUCGUCCAUC-5'** (SEQ ID NO: **10:sense)**
**DS04**
   **5' -GGUCCUCGUAGCGCACAAGCAGGUAGUdTdT-3'** (SEQ ID **NO: 11:antisense)**
   **3' -dTdTCCAGGAGCAUCGCGUGUUCGUCCAUCA-5'** (SEQ ID **NO: 12:sense)**
**DSOS**
   **5' -GUCCUcgUAgCGCACðAgCAGGUAGYGdTdT-3'** (SEQ ID NO: **13:ant**isense**)**
   **3' -dTdTCAGGAGCAUCGCGUGUUCGUCCAUCAC-5'** (SEQ ID NO: **14:sense)**

### siRNA (DS06 to DS10) having artificial bases

In one embodiment, the siRNA in the bispecific nucleic acid molecule of the present invention may comprise artificial bases. Specifically, as shown in DS06 to DS10, bases in the antisense strand marked with double underlines indicate mismatch base positions (where CHST2 and CHST4 do not match), for example, one or more positions of N selected from the group consisting of positions 3, 7, 11, 12, 13, 24 and 27 in SEQ ID NO: 15, positions 5, 8, 14, 16, 24, 26 and 27 in SEQ ID NO: 17, positions 9, 12, 18 and 20 in SEQ ID NO: 19, positions 8, 11, 17, 19 and 27 in SEQ ID NO: 21, and positions 7, 10, 16, 18 and 26 in SEQ ID NO: 23, and further all the selected positions of N may be artificial bases. The artificial bases may include, for example, but are not limited to, 2,6-diaminopurine and ribavirin, which may be 2'-OMe, 2'-MOE, or LNA-modified.

### [Chemical formula 2]

**DS06**
   5' -ACNGGGNCUCNNNCCAGGUCCUCNUANdTdT-3' (SEQ ID NO: 15 : ant i sense)
   3' -dTdTUGUCCCUGAGUUUGGUCCAGGAGUAUU-5' (SEQ ID NO: 16 : sense)
DS07
   5' -CCUCNUANCGCACNANCAGGUAGNGNNdTdT-3' (SEQ ID NO: 17:antisense)
   3' -dTdTGGAGUAUUGCGUGUUUGUCCAUCUCUU-5' (SEQ ID NO: 18 : sense)
DS08
   5' -AGGUCCUCNUANCGCACNANCAGGUAGdTdT-3' (SEQ ID NO: 19 : antisense)
   3' -dTdTUCCAGGAGUAUUGCGUGUUUGUCCAUC-5' (SEQ ID NO: 20 : sense)
DS09
   5' -GGUCCUCNUANCGCACNANCAGGUAGNdTdT-3' (SEQ ID NO: 21 : ant i sense)
   3' -dTdTCCAGGAGUAUUGCGUGUUUGUCCAUCU-5' (SEQ ID NO: 22 : sense)
DS10
   5' -GUCCUCNUANCGCACNANCAGGUAGNGdTdT-3' (SEQ ID NO: 23 : anti sense)
   3' -dTdTCAGGAGUAUUGCGUGUUUGUCCAUCUC-5' (SEQ ID NO: 24 : sense)

### Antisense oligonucleotides (SS01 to SS05):

In one embodiment, the bispecific nucleic acid molecules of the present invention may be antisense oligonucleotides having only the antisense strand as shown in SEQ ID NOs: 25 to 34. Similar to DS01 to DS05, bases in the antisense strand marked with double underlines indicate mismatch base positions (where CHST2 and CHST4 do not match), for example, one or more positions selected from the group consisting of positions 3, 7, 11, 12, 13, 24 and 27 in SEQ ID NO: 25, positions 5, 8, 14, 16, 24, 26 and 27 in SEQ ID NO: 26, positions 9, 12, 18 and 20 in SEQ ID NO: 27, positions 8, 11, 17, 19 and 27 in SEQ ID NO: 28, positions 7, 10, 16, 18 and 26 in SEQ ID NO: 29, positions 3, 7, 11, 12, 13, 24 and 27 in SEQ ID NO: 30, positions 5, 8, 14, 16, 24, 26 and 27 in SEQ ID NO: 31, positions 9, 12, 18 and 20 in SEQ ID NO: 32, positions 8, 11, 17, 19 and 27 in SEQ ID NO: 33, and positions 7, 10, 16, 18 and 26 in SEQ ID NO: 34, may be modified, for example, but are not limited to, 2'-OMe, 2'-MOE, or LNA modification. SS01 to SS05 have natural bases.

### [Chemical formula 3]

**SS01**
   **5' -ACGGGGGCUCGAGCCAGGUCCUCGUAG-3'** (SEQ ID NO: **25 : antisense)**
**SS02**
   **5' -CCUCGUAGCGCACAAGCAGGUAGUGGU-3'** (SEQ ID NO**: 26** : **antisense)**
**SS03**
   5' **-AGGUCCUCGUAGCGCACAAGCAGGUAG-3'** (SEQ ID NO: **27** : antisense**)**
**SS04**
   **5' -GGUCCUCGUAGCGCACAAGCAGGUAGU-3'** (SEQ ID NO**: 28** : antisense**)**
**SS05**
   **5' -GUCCUCGUAGCGCACAAGCAGGUAGUG-3'** (SEQ ID N**O: 29** : antisense**)**

### Antisense oligonucleotides (SS06 to SS10) having artificial bases

In one embodiment, the antisense oligonucleotides in the bispecific nucleic acid molecules of the present invention may comprise artificial bases. SS06 to SS10 are single-stranded nucleic acid sequences present only in the antisense strand, in particular, as shown in SS06 to SS10, bases in the antisense strand marked with double underlines indicate mismatch base positions (where CHST2 and CHST4 do not match), for example, one or more positions of N selected from the group consisting of positions 3, 7, 11, 12, 13, 24 and 27 in SEQ ID NO: 30, at positions 5, 8, 14, 16, 24, 26 and 27 in SEQ ID NO: 31, positions 9, 12, 18 and 20 in SEQ ID NO: 32, positions 8, 11, 17, 19 and 27 in SEQ ID NO: 33, and positions 7, 10, 16, 18 and 26 in SEQ ID NO: 34, and further all the selected positions of N may be replaced with artificial bases. The artificial bases may include, for example, 2,6-diaminopurine and ribavirin. Regardless of whether they are artificial bases or not, one or more to all of the bases may be, but are not limited to, 2'-OMe-modified, 2'-MOE-modified, or LNA-modified.

### [Chemical formula 4]

**SS06**
   5' -ACNGGGNCUCNNNCCAGGUCCUCNUAN-3' (SEQ ID NO:30 : ant isense)
**SS07**
   5' -CCUCNUANCGCACNANCAGGUAGNGNN-3' (SEQ ID NO: 31 : antisense)
**SS08**
   5' -AGGUCCUCNUANCGCACNANCAGGUAG-3' (SEQ ID NO: 32 : antisense)
**SS09**
   5' -GGUCCUCNUANCGCACNANCAGGUAGN-3' (SEQ ID NO: 33 : antisense)
**SS10**
   5' -GUCCUCNUANCGCACNANCAGGUAGNG-3' (SEQ ID NO: 34 : antisense)

In one aspect of the invention, in the bispecific nucleic acid molecule of the present invention, the bases in the antisense strand, particularly the bases at positions not matching between CHST2 and CHST4 may be substituted with artificial bases instead of natural bases. The artificial base may be any artificial base suitable as a pharmaceutical, preferably a universal base such as 2,6-diaminopurine, ribavirin, or inosine. Regardless of whether they are natural or artificial bases, one or more to all of the bases may be, but are not limited to, 2'-OMe-modified, 2'-MOE-modified, or LNA-modified.

In one aspect of the invention, the bispecific nucleic acid molecules of the present invention may be chemically modified to the artificial bases of the antisense strand. The chemical modification may be any modification suitable as a pharmaceutical, and may be selected from the group consisting of phosphonate modifications including methylphosphonate (MP), phosphorodithioate (PS2), methoxypropyl phosphonate (MPO), 5'-phosphorothioate (5'-PS), (S)-5'-C-methyl having phosphoate, peptide nucleic acid (PNA), phosphorothioate (PS, Rp isomer), phosphorothioate (PS, Sp isomer), 5'-(E)-vinylphosphole (5'-(E)-VP), and 5'-methylphosphonate (5'-MP); base modifications including pseudouridine (Ψ), 2'-thiouridine (s2U), N6'-methyladenosine (m⁶A), N-ethylpiperidine 7'-EAA triazole-modified adenine, N-ethylpiperidine 6'-triazole-modified adenine, 6'-phenylpyrrolo-cytosine (PhpC), 5'-methylcytidine (m5C), 5'-fluoro-2'-deoxyuridine, 2',4'-difluorotoluylribonucleoside (rF), and 5'-nitroindole; and ribose modifications including 2'-O-methyl (2'-OMe), 2'-deoxy-2'-fluoro (2'F), 2'-methyl-4-pyridinefluoro (2'-O-CH2Py(4)), PMO, 2'-O-methoxyethyl (2'-O-MOE), tricyclo-DNA (tcDNA), locked nucleic acid (LNA), 2'-arabino-fluoro (2'-Ara-F), 2'-O-benzyl, glycol nucleic acid (GNA), unlocked nucleic acid (UNA) and (S)-cEt-BNA, and may preferably be 2'-OMe.

In addition, in one aspect of the invention, the bispecific nucleic acid molecules of the present invention may be modified to enhance drug delivery, for example, but are not limited to, drug conjugates including antibody-siRNA conjugates, polymer-siRNA conjugates, GalNAc-siRNA conjugates and cholesterol-siRNA conjugates; lipid-based nanocarriers including liposomes, stealth liposomes, solid-lipid nanoparticles, stabilized nucleic acid lipid particles and lipid nanoparticles; polymer nanocarriers including degradable or non-differentiable polymer-based nanoparticles (chitosan, cyclodextrin, poly(ethyleneimine), poly(L-lysine) and poly(beta-amino-ester)), and dendrimers (poly(propyleneimine) and poly(amidoamine)); inorganic nanocarriers including silica nanoparticles, metal/metal oxide nanoparticles (Au and FexOy); and other modifications including carbon-based nanoparticles, hydrogels, quantum dots and natural ECVs (exosomes, larger ECVs).

The bispecific nucleic acid molecules of the present invention suppress the formation of lesions of inflammatory cell infiltration in inflammatory or allergic diseases as a single agent, so that the present invention provides a pharmaceutical composition for preventing or treating inflammatory or allergic diseases, comprising bispecific nucleic acid molecules targeting CHST2 and CHST4 as active ingredients.

The present invention provides a method for preventing or treating inflammatory or allergic diseases, comprising administering a bispecific nucleic acid molecule targeting CHST2 and CHST4, a pharmaceutical composition for use in preventing or treating inflammatory or allergic diseases, comprising a bispecific nucleic acid molecule targeting CHST2 and CHST4, a use of a bispecific nucleic acid molecule targeting CHST2 and CHST4 in the manufacture of a pharmaceutical composition for preventing or treating inflammatory or allergic diseases, and a method for manufacturing an agent for treating an inflammatory or allergic disease, comprising the step of using (formulating and/or mixing with a pharmaceutically or physiologically acceptable carrier) a bispecific nucleic acid molecule targeting CHST2 and CHST4.

The inflammatory or allergic diseases targeted for treatment or prevention in the present invention are inflammatory or allergic diseases associated with the expression of HEV-like blood vessels at the local site of inflammation where the bispecific nucleic acid molecules of the present invention exert therapeutic effects, specifically chronic inflammatory or allergic diseases, including, but are not limited to, Guillain-Barré syndrome, myasthenia gravis, inflammatory myopathy, chronic gastritis, chronic atrophic gastritis, autoimmune hepatitis, primary biliary cholangitis, inflammatory bowel disease, Crohn's disease, ulcerative colitis, autoimmune pancreatitis, Takayasu's arteritis, Goodpasture's syndrome, rapidly progressive glomerulonephritis, Graves' disease, Hashimoto's disease, primary hypothyroidism, idiopathic Addison's disease, type 1 diabetes, chronic discoid lupus erythematosus, localized scleroderma, pemphigus, pustular psoriasis, psoriasis vulgaris, epidermolysis bullosa, autoimmune optic neuropathy, chronic rheumatoid arthritis, arthritis, spondyloarthritis, systemic lupus erythematosus, scleroderma (systemic sclerosis), Sjogren's syndrome, polymyositis, dermatomyositis, vasculitis syndrome, mixed connective tissue disease, bronchial asthma, bronchiectasis, interstitial pneumonia, pulmonary fibrosis, allergic rhinitis, chronic rhinosinusitis, allergic conjunctivitis, chronic thyroiditis, transplant rejection, Warthin's tumor, prostatic hyperplasia, cutaneous pseudolymphoma, contact dermatitis, Th2 dominant diseases or type 2 inflammation, and atopic dermatitis. Also, the "treatment" in the present invention is not necessarily limited to cases with complete therapeutic effects, but may also be cases with partial effects.

The pharmaceutical composition of the present invention for treating inflammatory or allergic diseases may be mixed with a pharmaceutically or physiologically acceptable carrier, excipient, or diluent, etc., and administered orally or parenterally. For oral formulation, it may be in the form of granules, powders, tablets, capsules, solutions, emulsions, or suspensions, etc. For parenteral formulation, it may select injections or infusions. Injections may include subcutaneous injections, intramuscular injections, intraperitoneal injections, intracranial injections, or intranasal injections, etc. Formulation techniques to make the above-mentioned forms so as to comprise the pharmaceutical composition of the present invention as the main ingredient are well known.

For example, tablets for oral administration can be manufactured by mixing the pharmaceutical composition of the present invention for restoring tissue integrity with excipients, disintegrants, binders, and lubricants, etc., and then compression shaping. As the excipients, lactose, starch, or mannitol, etc., are generally used. As the disintegrants, calcium carbonate or calcium carboxymethylcellulose, etc., are generally used. As the binders, gum arabic, carboxymethyl cellulose, or polyvinylpyrrolidone are used. As the lubricants, talc or magnesium stearate, etc., are known in the art.

The tablets of the pharmaceutical composition for treating inflammatory or allergic diseases of the present invention may be coated with a known coating for masking or to make them enteric coated formulation. As the coating agent, ethyl cellulose, polyoxyethylene glycol, etc., may be used.

Also, the injections can be obtained by dissolving with a suitable dispersant, dissolving in a dispersion medium or dispersing the pharmaceutical composition for restoring tissue integrity of the present invention as the main ingredient. Depending on the selection of the dispersion medium, the formulation can be either of the solvent types of aqueous solvent and oil-based solvent. For aqueous solvent, distilled water, physiological saline, or Ringer's solution, etc., are used as the dispersion medium. For oil-based solvent, various kinds of vegetable oils or propylene glycol, etc., can be used as the dispersion medium. At this time, preservatives such as parabens may be added if necessary. Also, to the injections, known isotonic agents, such as sodium chloride or glucose may also be added. Further, analgesics such as benzalkonium chloride or procaine hydrochloride may be added.

When using the pharmaceutical composition for preventing or treating inflammatory or allergic diseases of the present invention as a gene therapy agent, there may be included a method of directly administering the pharmaceutical composition for preventing or treating inflammatory or allergic diseases of the present invention by injection or a method of administering a vector into which the nucleic acid is incorporated. As the above-mentioned vectors, there may be included adenovirus vectors, adeno-associated virus vectors, herpesvirus vectors, vaccinia virus vectors, retrovirus vectors, lentivirus vectors, etc., and using these viral vectors enables efficient administration.

In addition, it is also possible to introduce the pharmaceutical composition for preventing or treating inflammatory or allergic diseases of the present invention into phospholipid vesicles such as liposomes and administer these vesicles. Vesicles comprising siRNA are introduced into predetermined cells using the lipofection method. And the obtained cells are then administered systemically, for example, intravenously or intra-arterially, etc.

In addition, the present invention also provides a method for improving lesions in a subject, comprising the step of administering a pharmaceutical composition for preventing or treating inflammatory or allergic diseases of the present invention to an individual (for example, a patient) or to the inflamed tissue thereof. The individual to be a subject of the present invention is not particularly limited to any organism capable of developing changes in inflammatory or allergic lesions, but is preferably mammals including humans, dogs or cats. Administration to the subject may be performed by methods known to a person skilled in the art, such as oral administration, topical application to the skin, spraying, intradermal administration or subcutaneous administration, or intravenous injection. Systemic administration or direct local administration into the inflamed tissue is possible. Also, commercially available gene delivery kits may also be used to introduce the bispecific nucleic acid molecules of the present invention into the target cells, tissues or organs.

The pharmaceutical composition for preventing or treating inflammatory or allergic diseases of the present invention is administered to mammalian subjects, including humans, at a required amount (effective amount) within the range of dosages considered to be safe. The dosage of the pharmaceutical composition for preventing or treating inflammatory or allergic diseases of the present invention can be appropriately determined based on the judgment of a person skilled in the art (physician or veterinarian), considering factors such as the type of dosage form, administration method, age and weight of the subject, and the subject's symptoms. As one example, although it varies depending on age, sex, symptoms, route of administration, frequency of administration, and dosage form, for example, the dosage for local administration of the bispecific nucleic acid molecule of the present invention is approximately 1 pM to 10,000 nM per dose once daily, administered as a single dose, at intervals ranging from one week to one month (for example, weekly, biweekly, or monthly), or daily.

All prior art documents cited herein are incorporated by reference into the present specification.

### EXAMPLES

Hereinafter, the present invention will be described in more detail using Examples. However, the technical scope of the present invention is not limited to these examples.

### <Example 1> Knockdown effect of bispecific nucleic acids (10 nucleic acids of DS01 to DS10; available from BioSpring) targeting human CHST2 and CHST4 on target mRNA (CHST2 mRNA and CHST4 mRNA):

A cell line simultaneously expressing human CHST2 and CHST4 (dual transfectant) was prepared and cultured in vitro, and the suppressing effects on CHST2/CHST4 mRNA expression of 10 synthetic double stranded RNAs (dsRNAs, nucleic acid compound numbers: DS01 to DS10), standardized to a concentration of 50 nM, were examined by quantitative RT-PCR. As a result, all 10 designed and synthesized dsRNAs simultaneously showed suppressing effects on both mRNAs of CHST2/CHST4. Control: Vehicle (physiological saline) was used as the negative control, and PC: A cocktail of CHST2 siRNA + CHST4 siRNA was used as the positive control.

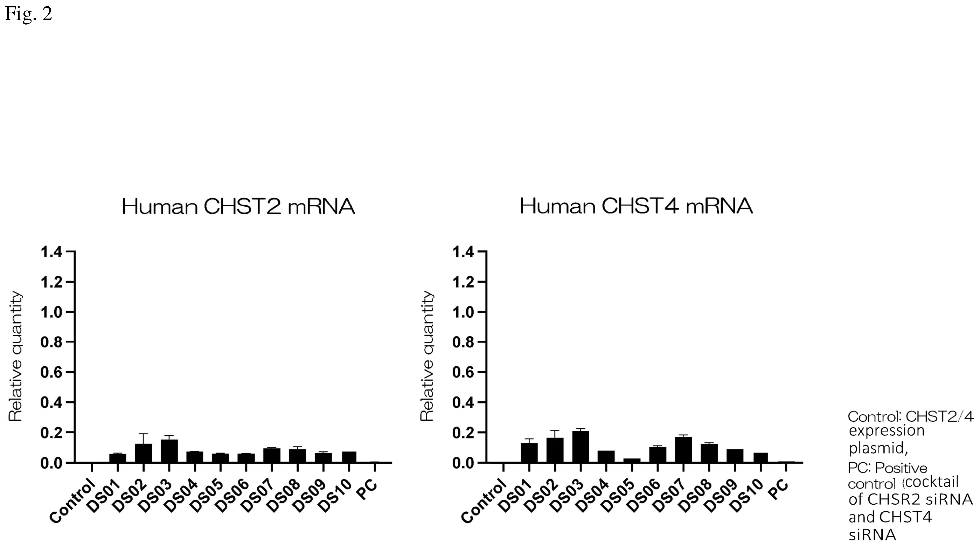

The results are shown in Fig. 2.

### <Example 2> Binding affinity of bispecific nucleic acids (5 nucleic acids of DS06 to DS10) targeting Human CHST2:

The stability of the binding complexes between the antisense strands (single-stranded RNA, SS06 to SS10) of the dsRNAs, DS06 to 10 used in Example 1, and human CHST2 mRNA was analyzed by measuring the double-helix melting temperature (Tm). As a result, all SSs (antisense strands of the bispecific nucleic acids) showed strong affinity, and it was found that SS06 had the strongest binding affinity to human CHST2 mRNA.

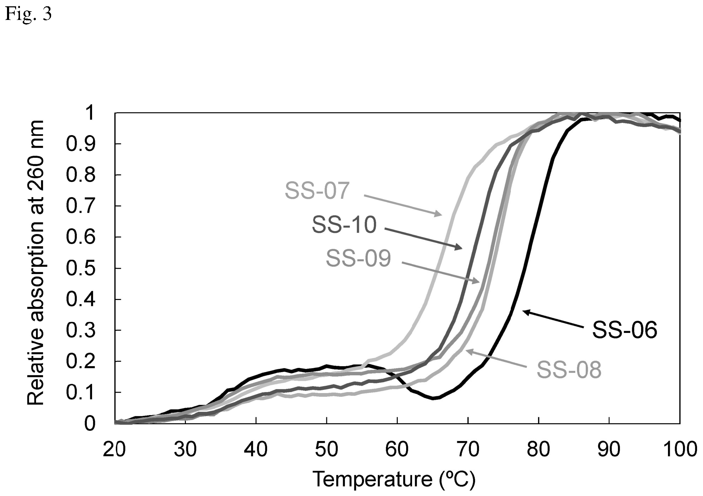

The results are shown in Fig. 3.

### <Example 3> Binding affinity of bispecific nucleic acids (5 nucleic acids of DS06 to DS10) targeting Human CHST4:

The stability of the binding complexes between the antisense strands (single-stranded RNA, SS06 to SS10) of the dsRNAs, among DS06 to DS10 used in Example 1 and human CHST4 mRNA was analyzed by measuring the double-helix melting temperature (Tm). As a result, all SSs (antisense strands of the bispecific nucleic acids) showed strong affinity, and it was found that SS06 had the strongest binding affinity to human CHST4 mRNA.

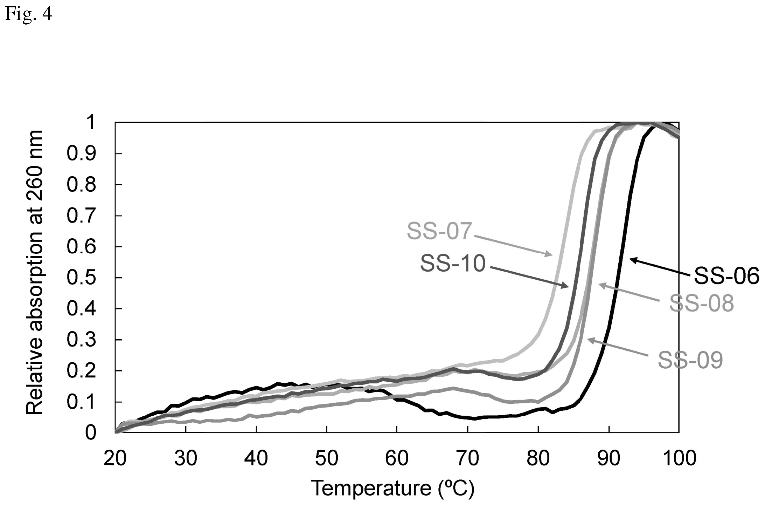

The results are shown in Fig. 4.

### <Example 4> Dose-dependent knockdown effect of bispecific nucleic acids (2 nucleic acids of DS06 and DS10) targeting human CHST2 and CHST4 on their respective target mRNAs (CHST2 mRNA and CHST4 mRNA):

Using the same method as in Example 1, a cell line (dual transfectant) simultaneously expressing human CHST2/CHST4 was prepared and cultured in vitro, and the dose-dependent suppressing effect of DS06 or DS10 on CHST2/CHST4 mRNA expression was examined by quantitative RT-PCR. As a result, both DS06 and DS10 simultaneously showed suppressing effects on two mRNAs of CHST2/CHST4 starting from a low concentration of 50 nM. Control: Vehicle (physiological saline) was used as the negative control and PC: A cocktail of CHST2 siRNA + CHST4 siRNA was used as the positive control.

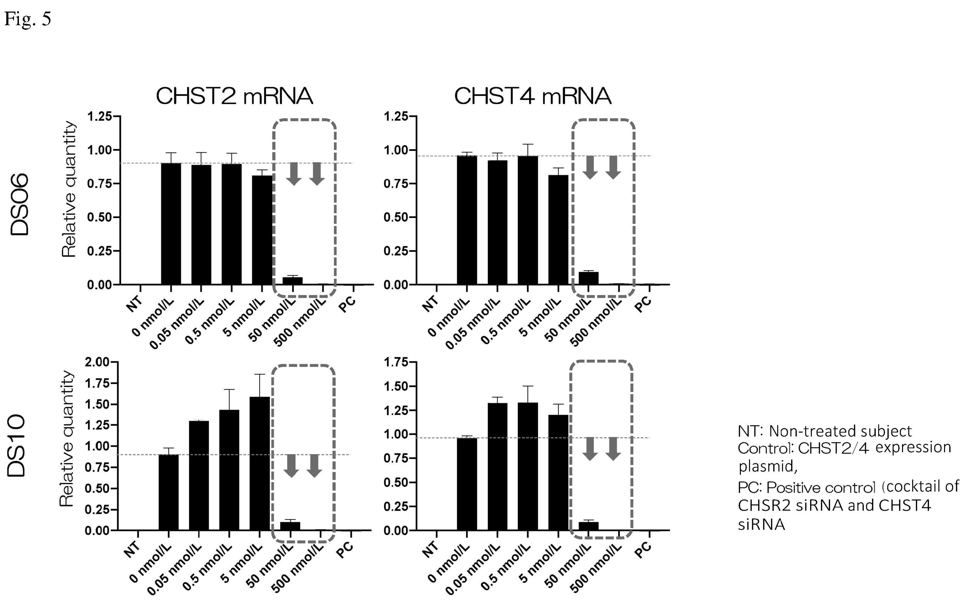

The results are shown in Fig. 5.

### <Example 5> Dose-dependent expression suppressing effect of bispecific nucleic acids (2 nucleic acids of DS06 and DS10) targeting human CHST2 and CHST4 on MECA-79:

Using the same method as in Example 1, a cell line (dual transfectant) simultaneously expressing human CHST2/CHST4 was prepared and cultured in vitro, and the dose-dependent suppressing effect of DS06 or DS10 on MECA-79 epitope expression was examined by a flow cytometer (FACS). As a result, both DS06 and DS10 showed suppressing effect of MECA-79 expression exceeding the positive control, and the dose-dependent suppressing effect was more remarkable for DS06. Control: Vehicle (physiological saline) was used as the negative control and PC: A cocktail of CHST2 siRNA + CHST4 siRNA was used as the positive control.

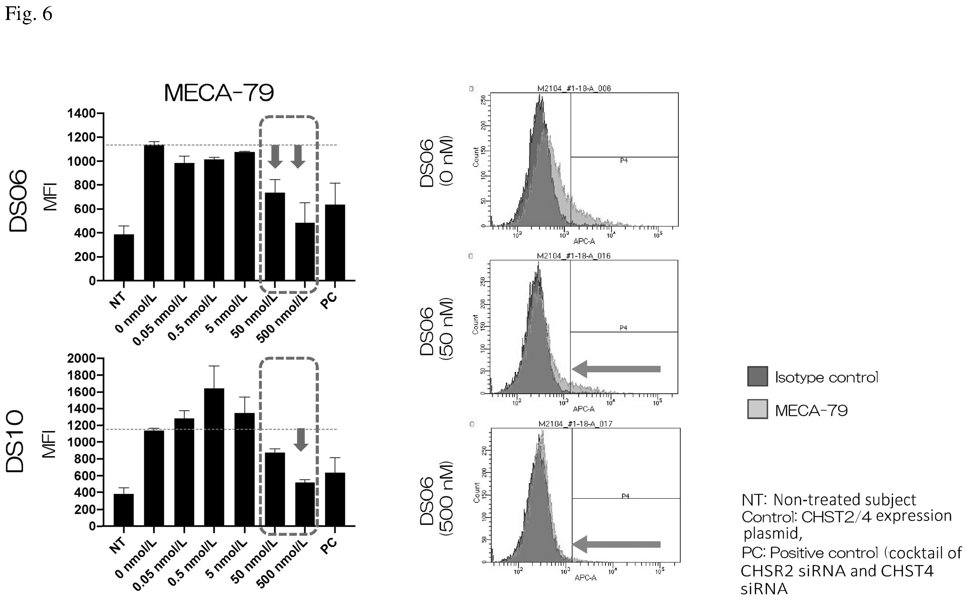

The results are shown in Fig. 6.

### <Example 6> Effect of bispecific nucleic acid (DS06) targeting human CHST2 and CHST4 on interferon response gene (IFITM1) expression:

Using the same method as Example 1, a cell line (dual transfectant) simultaneously expressing human CHST2/CHST4 was prepared and cultured in vitro, and whether DS06 increased the expression of the interferon-response gene IFITM1 mRNA, i.e., whether it could induce a non-specific interferon response was examined by quantitative RT-PCR. As a result, DS06 did not induce an increase in IFITM1 mRNA expression even at the maximum concentration of 500 nM. On the other hand, Poly I:C was used as a positive control for inducing an interferon response, and did indeed elicit a strong interferon response in this experimental system.

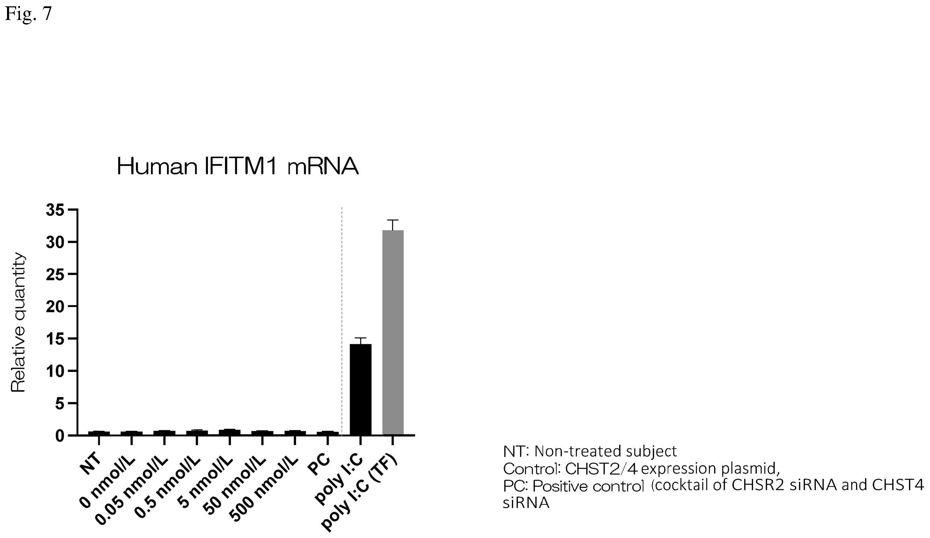

The results for DS06 are shown in Fig. 7.

### <Example 7> Effects of bispecific nucleic acids (2 nucleic acids of DS06 and DS10) targeting human CHST2 and CHST4 on TLR responses:

Whether DS06 induces non-specific inflammation mediated by Toll-like receptors (TLRs) was analyzed by the response effects on human TLR2, 3, 4, 5, 7, 8 and 9. As a result, DS06 did not induce the above-mentioned TLR responses even at the high concentration of 1 µM. Control: Substances known to induce each TLR response were used as positive controls.

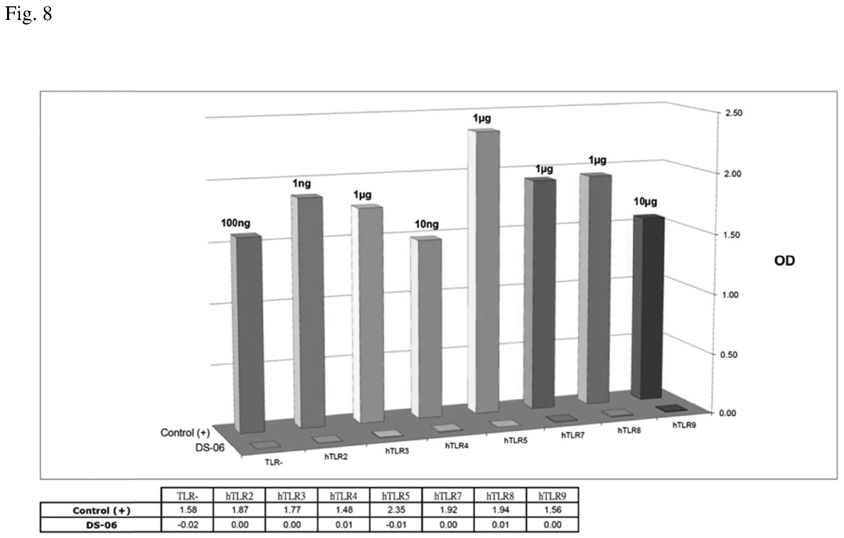

The results are shown in Fig. 8.

### <Example 8> Skin permeation test using artificial skin of DS-06:

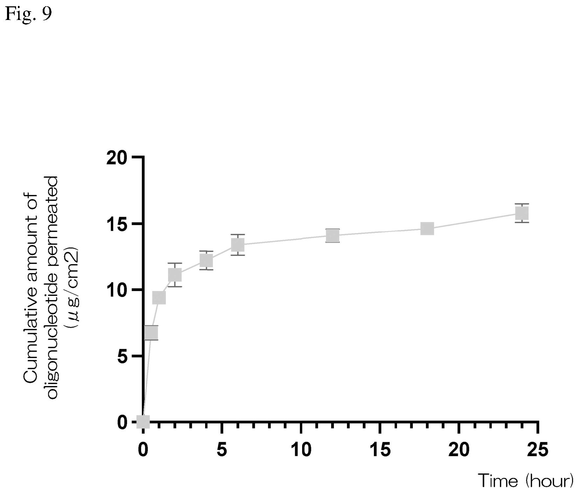

A DS-06-containing cream base composition was prepared and applied to the surface of artificial skin (Strat M) using a Franz cell, and the cumulative total amount of the nucleic acid permeated over time (0, 0.5, 1, 2, 4, 6, 12, 18 and 24 hours) was measured by HPLC. As shown in Fig. 9, DS06 showed excellent permeability.

The results are shown in Fig. 9.

### <Example 9> Effect of bispecific nucleic acid (DS06) targeting human CHST2 and CHST4 on mouse atopic dermatitis:

Based on a previous report (Sasakawa T, Higashi Y, Sakuma S, et al. Atopic dermatitis-like skin lesions induced by topical application of mite antigens in NC/Nga mice. Int Arch Allergy Immunol. 126: 239-247, 2001), after administering mite antigens to the ear auricle of NC/Nga mice (7 weeks old, male) to induce dermatitis, a DS-06-containing cream, a negative control cream, and tacrolimus ointment as a positive control were applied once daily for 20 days from day 11 to 30, and the mice were slaughtered on day 31 to evaluate skin lesions. Results are shown by HE staining findings. Compared to the control cream groups, the DS06 cream group clearly showed a marked reduction in inflammatory cell infiltration accompanied by thickening extending into the dermis and deeper layers.

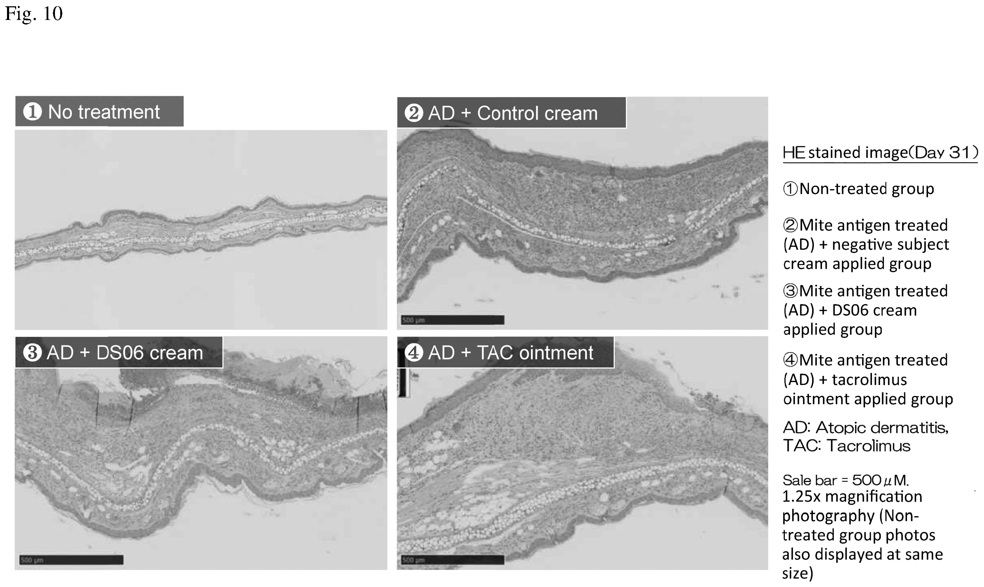

The results are shown in Fig. 10.

### <Example 10> Effect of bispecific nucleic acid (DS06) targeting human CHST2 and CHST4 on mouse atopic dermatitis (HEV-like blood vessels):

In the test of Example 9, the results of quantifying the extent of HEV-like blood vessel emergence as MECA-79-positive area (%) are shown by immunostaining with MECA-79 antibody. In the dermatitis model, MECA-79-positive vessels significantly increased, but application of DS06 cream significantly reduced the MECA-79-positive area compared to the control group.

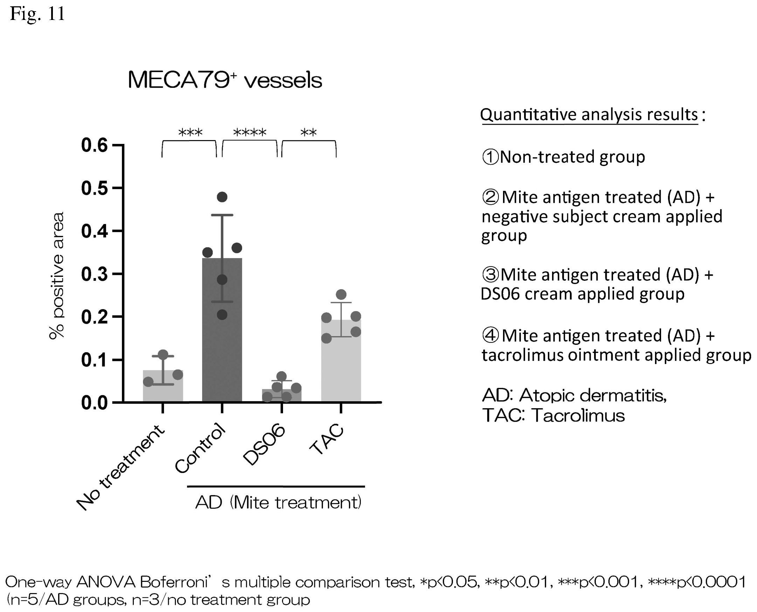

The results are shown in Fig. 11.

### <Example 11> Effect of bispecific nucleic acid (DS06) targeting human CHST2 and CHST4 on mouse atopic dermatitis (T lymphocytes):

In the test of Example 9, the results of quantifying the extent of lymphocyte infiltration into the dermis as CD4-positive area (%) are shown by immunostaining with anti-CD4 antibody. In the dermatitis model, CD4⁺ T cells significantly increased, but application of DS06 cream significantly reduced the CD4⁺ T cell positive area compared to the control group.

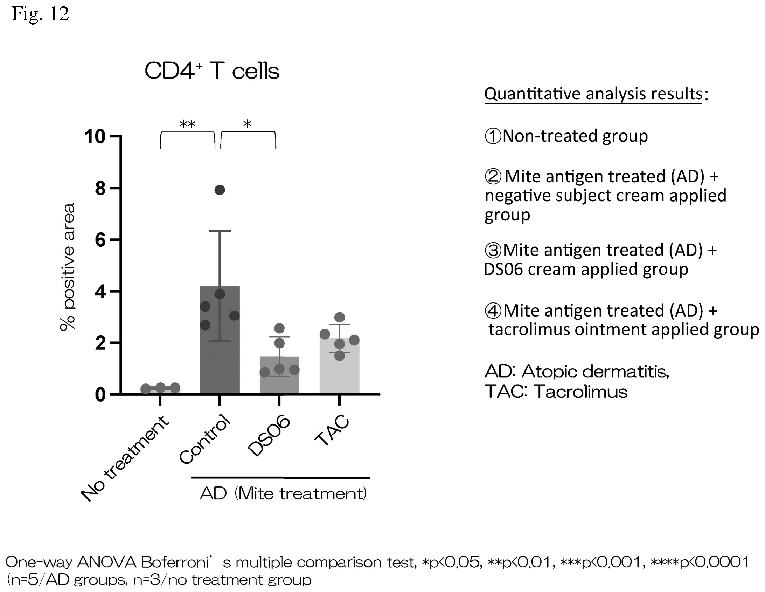

The results are shown in Fig. 12.

### <Example 12> Effect of bispecific nucleic acid (DS06) targeting human CHST2 and CHST4 on mouse atopic dermatitis (Activated T lymphocytes):

In the test of Example 9, the results of quantifying the extent of activated lymphocyte infiltration into the dermis as CD69-positive area (%) are shown by immunostaining with anti-CD69 antibody, which is also a marker for Resident memory T cells. In the dermatitis model, CD69⁺ cells significantly increased, but application of DS06 cream significantly reduced the CD69⁺ cell positive area compared to the control group.

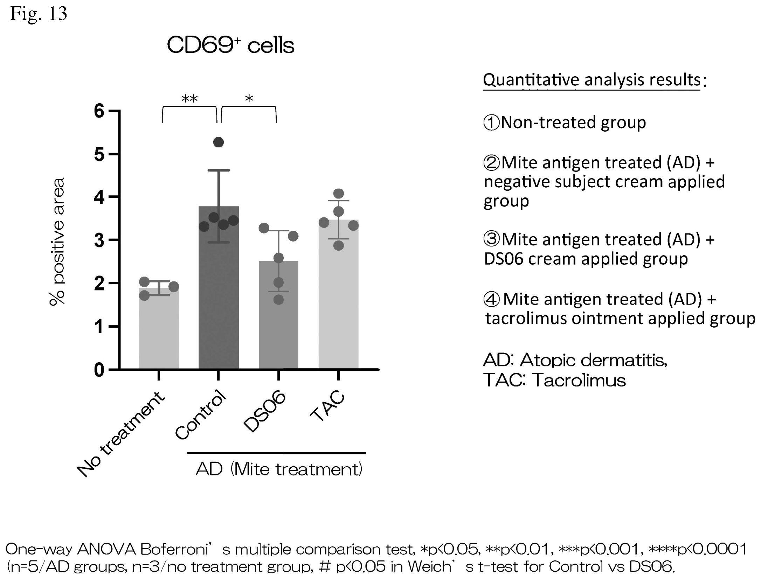

The results are shown in Fig. 13.

### <Example 13> Effect of bispecific nucleic acid (DS06) targeting human CHST2 and CHST4 on mouse atopic dermatitis (Regulatory T lymphocytes):

In the test of Example 9, the results of quantifying the extent of regulatory T cell (Treg) infiltration into the dermis as FoxP3-positive area (%) are shown by immunostaining with Treg marker anti-FoxP3 antibody. In the dermatitis model, FoxP3⁺ Tregs tended to decrease, but application of DS06 cream tended to significantly increase FoxP3⁺ Treg positive area compared to the control group. It was maintained with similar levels to those in normal mice. On the other hand, tacrolimus ointment, planned to use as a positive control, showed a rather tendency toward reduced Tregs, but DS06 cream significantly maintained Treg infiltration compared to the tacrolimus group.

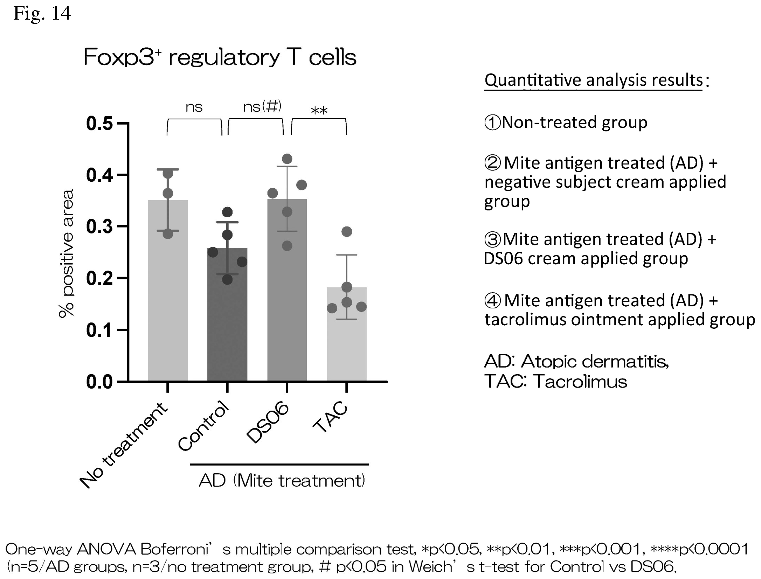

The results are shown in Fig. 14.

### <Example 14> Effect of bispecific nucleic acid (DS06) targeting human CHST2 and CHST4 on mouse atopic dermatitis (IL-13 and IL-33):

In the test of Example 9, the results where RNA was purified from skin tissue and the expression levels of Type 2 cytokines were quantified by RT-PCR are shown. In the dermatitis model, IL-13 expression and IL-33 expression were increased, but application of DS06 cream showed a tendency to significantly decrease the expression of IL-13 and IL-33 compared to the control group.

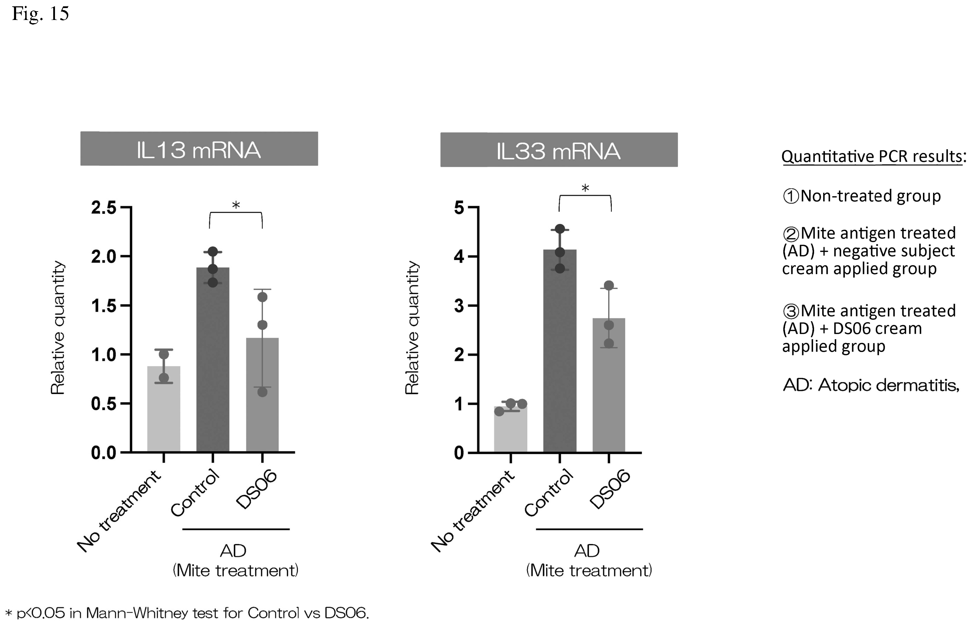

The results are shown in Fig. 15.

### <Example 15> Effect of bispecific nucleic acid (DS06) targeting human CHST2 and CHST4 on mouse allergic rhinitis (HEV-like blood vessels and T lymphocytes):

Based on a previously report (Ohmichi Y, Hirakawa J, Imai Y, et al. Essential role of peripheral node addressin in lymphocyte homing to nasal-associated lymphoid tissues and allergic immune responses. J Exp Med. 208: 1015-25, 2011), allergic rhinitis was induced in C57BL/6J mice (6 weeks old, male) by intraperitoneal administration of a mixed solution of OVA (ovalbumin) and CT (cholera toxin) at weekly intervals for a total of three times (day 0, day 7 and day 14). During the period from day 8 to day 19, DS06 solution and physiological saline as a negative control were administered intranasally five times per week (total of 10 administrations), and animals were slaughtered on day 21 to evaluate nasal tissue lesions.

The results of quantifying the extent of HEV-like vessel emergence in the Nasal-associated lymphoid tissue (NALT) as the MECA-79-positive area (%) are shown by immunostaining with MECA-79 antibody, as in the method of Example 10. In the rhinitis model, intranasal administration of DS06 significantly reduced the MECA-79-positive area compared to the control group. Furthermore, the results of quantifying the extent of lymphocyte infiltration as the CD4-positive area (%) by immunostaining with anti-CD4 antibody, as in the method of Example 11. In the rhinitis model, intranasal administration of DS06 significantly reduced the CD4⁺ T cell-positive area compared to the control group.

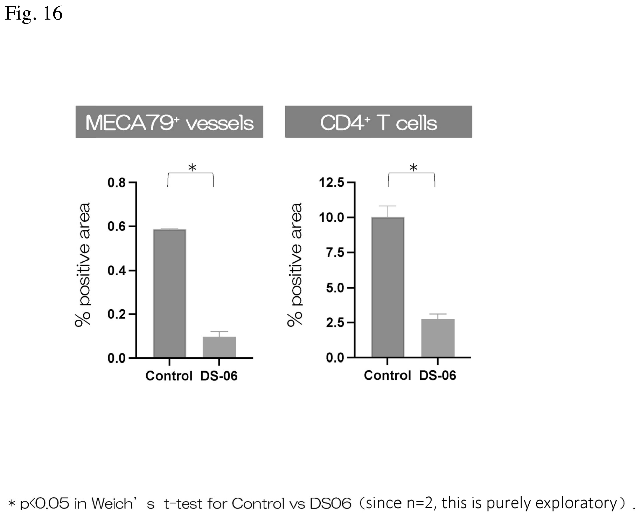

The results are shown in Fig. 16.

### INDUSTRIAL APPLICABILITY

The bispecific nucleic acid molecules targeting carbohydrate sulfotransferases CHST2 and CHST4 of the present invention have been shown to inhibit the expression of HEV-like blood vessels that induce recruitment of leukocytes to sites of inflammatory infiltration in inflammatory or allergic diseases, and in particular to alleviate the pathogenesis and functional impairment of chronic lesions.

From these results, the bispecific nucleic acid molecules of the present invention are shown to be useful as pharmaceutical compositions for preventing or treating inflammatory or allergic diseases.

## Claims

1. A bispecific nucleic acid molecule comprising an antisense strand having a sequence that specifically binds to CHST2 and also specifically binds to CHST4.

2. The bispecific nucleic acid molecule according to Claim 1, which is an siRNA or an antisense oligonucleotide.

3. The bispecific nucleic acid molecule according to Claim 2, wherein the bispecific nucleic acid molecule is the siRNA having a sequence shown in SEQ ID NOs: 5 and 6, SEQ ID NOs: 7 and 8, SEQ ID NOs: 9 and 10, SEQ ID NOs: 11 and 12, or SEQ ID NOs: 13 and 14, or is the antisense oligonucleotide having any of SEQ ID NOs: 25 to 29.

4. The bispecific nucleic acid molecule according to Claim 2 or 3, which comprises an artificial base.

5. The bispecific nucleic acid molecule according to Claim 4, which comprises a chemically modified artificial base.

6. A pharmaceutical composition comprising the bispecific nucleic acid molecule according to any one of Claims 1 to 5.

7. The pharmaceutical composition according to Claim 6 for preventing or treating an inflammatory or allergic disease.

8. Use of the bispecific nucleic acid molecule according to any one of Claims 1 to 5 for the manufacture of a pharmaceutical composition for preventing or treating an inflammatory or allergic disease.

9. A method for preventing or treating a patient with an inflammatory or allergic disease, comprising administering the pharmaceutical composition according to Claim 6 or 7 to a patient in need of such treatment.
